# EUROPEAN PATENT APPLICATION

(11) **EP 4 129 967 A1**
(43) Date of publication of application: **08.02.2023**
(21) Application number: 21779691.1
(22) Date of filing: 19.01.2021
(51) Int. Cl.: C07C 51/08, C07B 61/00, C07C 51/06, C07C 59/06

(54) **METHOD FOR PRODUCING GLYCOLIC ACID SALT AND METHOD FOR PRODUCING GLYCOLIC ACID**

(30) Priority: 31.03.2020 JP 2020063561
(71) Applicant: SUMITOMO CHEMICAL COMPANY, LIMITED, Tokyo 103-6020 (JP)
(72) Inventor: IKEGUCHI, Masayuki, Niihama-shi, Ehime 792-8521 (JP); ISHIHARA, Takahiro, Osaka-shi, Osaka 554-8558 (JP)
(74) Representative: Global IP Europe Patentanwaltskanzlei
(86) International application number: PCT/JP2021/001623
(87) International publication number: WO 2021/199589

(57) **Abstract**

A glycolic acid salt that can be a raw material (intermediate product) for producing glycolic acid is more efficiently produced by a simple process. A method for producing a glycolic acid salt includes step (1) of reacting at least one compound selected from the group consisting of glycolonitrile and glycolamide with water in the presence of a metal oxide containing 50% by mass or more of at least one element selected from the group consisting of a rare earth element, a group 4 element of the periodic table, and a group 12 element of the periodic table, and a base to obtain a glycolic acid salt.

## Description

### TECHNICAL FIELD

The present invention relates to a method for producing a glycolic acid salt and a method for producing glycolic acid.

### BACKGROUND ART

Glycolic acid is used as, for example, a detergent, a leather tanning agent, or a chelating agent, and also as a raw material for cosmetics and pharmaceuticals. As a method for producing such glycolic acid, for example, a method is known in which glycolonitrile is produced using formaldehyde as a starting material, a glycolic acid salt is produced from glycolonitrile using an enzyme derived from a microorganism, and glycolic acid is produced from the glycolic acid salt (see Patent Document 1).

### PRIOR ART DOCUMENTS

### PATENT DOCUMENTS

Patent Document 1: WO-A-2006-126626

### SUMMARY OF THE INVENTION

### PROBLEMS TO BE SOLVED BY THE INVENTION

However, according to the method for producing glycolic acid according to Patent Document 1, in obtaining a glycolic acid salt, an enzyme reaction is performed using a suspension solution of a microorganism or a treated product of a microorganism (a disrupted product of a microorganism, an enzyme separated and extracted from a disrupted product of a microorganism, an immobilized microorganism, or a treated product on which an enzyme separated and extracted from a microorganism is immobilized). Therefore, there is a problem that an extremely complicated step of removing a microorganism or a treated product of a microorganism used in the reaction from the reaction system or the reaction product is essential.

### MEANS FOR SOLVING THE PROBLEMS

The present inventors made intensive studies in view of the above problems, and as a result, have found that the above problems can be solved by using a specific metal oxide catalyst, and have completed the present invention. That is, the present invention provides the following [1] to [9].
[1] A method for producing a glycolic acid salt, including step (1) of reacting at least one compound selected from the group consisting of glycolonitrile and glycolamide with water in the presence of a metal oxide containing 50% by mass or more of at least one element selected from the group consisting of a rare earth element, a group 4 element of the periodic table, and a group 12 element of the periodic table, and a base to obtain a glycolic acid salt.
[2] The method for producing a glycolic acid salt according to [1], further including
   step (2) of reacting formaldehyde with at least one compound selected from the group consisting of hydrogen cyanide and a cyanide in the presence of at least one compound selected from the group consisting of a metal oxide and a base to obtain glycolonitrile
   before the step (1) is performed.
[3] The method for producing a glycolic acid salt according to [2], in which the steps (1) and (2) are performed using a single reactor.
[4] The method for producing a glycolic acid salt according to any one of [1] to [3], in which the metal oxide is at least one metal oxide selected from the group consisting of cerium oxide, zinc oxide, zirconium oxide, and a composite oxide in which two or more of cerium oxide, zinc oxide, and zirconium oxide are combined.
[5] The method for producing a glycolic acid salt according to any one of [1] to [4], in which the metal oxide is cerium oxide.
[6] The method for producing a glycolic acid salt according to any one of [1] to [5], in which the base is at least one base selected from the group consisting of an alkali metal hydroxide and an alkaline earth metal hydroxide.
[7] The method for producing a glycolic acid salt according to any one of [1] to [6], in which the base is potassium hydroxide or sodium hydroxide.
[8] The method for producing a glycolic acid salt according to [2], in which in the step (2), the formaldehyde is reacted with the hydrogen cyanide.
[9] A method for producing glycolic acid, including step (3) of obtaining glycolic acid from a glycolic acid salt obtained by the method for producing a glycolic acid salt according to any one of [1] to [8].

### EFFECT OF THE INVENTION

The method for producing a glycolic acid salt according to the present invention makes it possible to efficiently produce a glycolic acid salt that can be a raw material (intermediate product) for producing glycolic acid by a simpler process.

### MODE FOR CARRYING OUT THE INVENTION

Hereinafter, an embodiment according to the present invention will be specifically described. The present invention is not limited by the following description.

A method for producing a glycolic acid salt according to the present embodiment includes step (1) of reacting at least one compound selected from the group consisting of glycolonitrile and glycolamide with water in the presence of a metal oxide containing 50% by mass or more of at least one element selected from the group consisting of a rare earth element, a group 4 element of the periodic table, and a group 12 element of the periodic table, and a base to obtain a glycolic acid salt.

### 1. Step (1)

As described above, step (1) is a step of reacting at least one compound selected from the group consisting of glycolonitrile and glycolamide with water using a "metal oxide containing 50% by mass or more of at least one element selected from the group consisting of a rare earth element, a group 4 element of the periodic table, and a group 12 element of the periodic table" as a catalyst in the presence of a base to obtain a glycolic acid salt as a reaction product.

More specifically, for example, as indicated in the following scheme, step (1) can be a step of reacting glycolonitrile and/or glycolamide as a substrate (intermediate product) with water using, as a catalyst, cerium oxide (CeO₂) which is a metal oxide containing 50% by mass or more of cerium as a rare earth element in the presence of potassium hydroxide as a base to obtain a glycolic acid salt represented by the following formula as a reaction product.

In particular, according to such a mode, the yield of a glycolic acid salt to be produced can be remarkably increased.

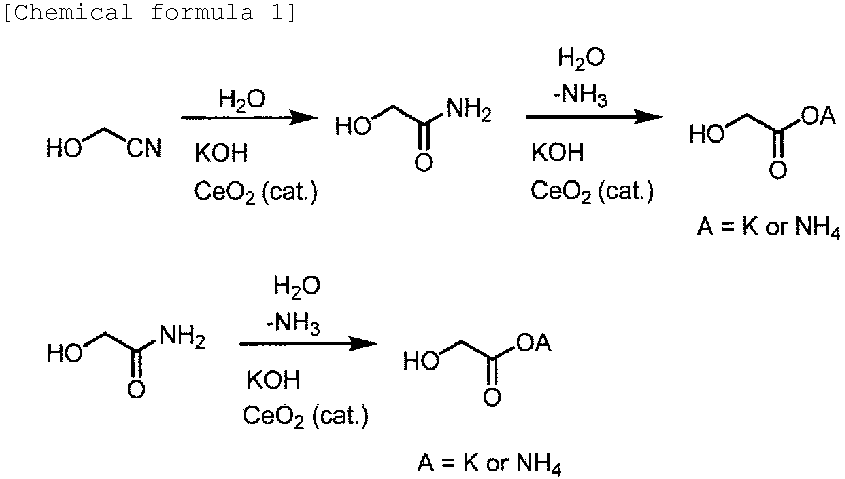

Preferred examples of a glycolic acid salt that can be produced by the method for producing a glycolic acid salt of the present embodiment include ammonium glycolic acid salt, potassium glycolic acid salt, and sodium glycolic acid salt.

Hereinafter, the metal oxide and the base used in step (1) and reaction conditions will be described more specifically.

### (i) Metal oxide

The metal oxide used in step (1) functions as a catalyst. The property of the metal compound is not particularly limited. The metal compound may be, for example, a powdery material, or may be a compact molded into an arbitrary suitable specific shape determined in consideration of a device and conditions applied to the production method of the present embodiment.

The metal oxide contains 50% by mass or more of at least one element selected from the group consisting of a rare earth element (scandium (Sc), yttrium (Y), and lanthanoids), a group 4 element of the periodic table (titanium (Ti), zirconium (Zr), and hafnium (Hf)), and a group 12 element of the periodic table (zinc (Zn), cadmium (Cd), and mercury (Hg)).

The rare earth element that can be contained in the metal oxide is preferably cerium or lanthanum, and more preferably cerium from a viewpoint of the yield of a glycolic acid salt.

The group 4 element of the periodic table that can be contained in the metal oxide is preferably zirconium or titanium, and more preferably zirconium.

The group 12 element of the periodic table that can be contained in the metal oxide is preferably zinc.

Specific examples of the metal oxide include cerium oxide and lanthanum oxide as oxides of a rare earth element, zirconium oxide and titanium oxide as oxides of a group 4 element of the periodic table, and zinc oxide as an oxide of a group 12 element of the periodic table.

The metal oxide is preferably cerium oxide, zirconium oxide, or zinc oxide from a viewpoint of the yield of a glycolic acid salt, and is more preferably cerium oxide because the yield of a glycolic acid salt can be remarkably increased.

The metal oxide may be at least one metal oxide selected from the group consisting of cerium oxide, zinc oxide, zirconium oxide, and a composite oxide in which two or more of cerium oxide, zinc oxide, and zirconium oxide are combined.

Specific examples of the composite oxide which is a metal oxide include a composite oxide of cerium oxide and zirconium oxide, and a composite oxide of cerium oxide and zinc oxide.

A lower limit of the content of the rare earth element, the group 4 element of the periodic table, or the group 12 element of the periodic table that can be contained in the metal oxide is preferably 50% by mass or more, more preferably 60% by mass or more, and still more preferably 70% by mass or more from a viewpoint of the yield of a glycolic acid salt. An upper limit of the content is preferably 90% by mass or less, more preferably 86% by mass or less, and still more preferably 82% by mass or less. Here, the content of an element contained in the metal oxide is a ratio of the mass of a certain element among the substances constituting the metal oxide with respect to the mass of the metal oxide. The content of an element can be measured by, for example, an element analysis method such as high-frequency inductively coupled plasma emission spectroscopy or X-ray fluorescence analysis.

### (ii) Base

Examples of the base used in step (1) include ammonia, a water-soluble salt of an alkali metal, and a water-soluble salt of an alkaline earth metal. The base is preferably a hydroxide of an alkari metal (for example, sodium hydroxide or potassium hydroxide) or a carbonate of an alkari metal (for example, sodium carbonate or potassium carbonate), or a hydroxide of an alkaline earth metal (for example, magnesium hydroxide or calcium hydroxide) or a carbonate of an alkaline earth metal (for example, magnesium carbonate or calcium carbonate), and more preferably sodium hydroxide or potassium hydroxide.

In step (1), one kind of base may be used singly, or two or more kinds thereof may be used in combination.

### (iii) Reaction conditions

The use amount of glycolonitrile and/or glycolamide is usually in a range of 0.1 to 10,000,000 parts by mass, and preferably in a range of 10 to 1,000,000 parts by mass with respect to 100 parts by mass of the metal oxide described above.

The use amount of the base is usually 0.001 to 100000 parts by mass, and preferably 0.01 to 10000 parts by mass with respect to 100 parts by mass of the metal oxide. The molar use amount of the base is usually in a range of 0.0001 to 1000, and preferably in a range of 0.001 to 100 with respect to the molar use amount of glycolonitrile and/or glycolamide.

Reaction time in step (1) only needs to be determined in consideration of conditions such as a catalyst to be used and reaction temperature. The reaction time is preferably in a range of 0.1 to 50 hours, more preferably in a range of 0.3 to 10 hours, and still more preferably in a range of 0.3 to 5 hours. In particular, when cerium oxide is used as a catalyst, the reaction time can be shorter, and a high yield can be achieved even in a short reaction time.

Reaction temperature is preferably in a range of 20 to 100°C, and more preferably in a range of 40 to 90°C. When the reaction temperature is set to be higher within the above range, the yield may be further improved.

Reaction pressure is preferably in a range of 0 to 1.0 MPa/G (here, "/G" means a gauge pressure, and the same applies hereinafter).

Examples of a reactor applicable to step (1) include a batch type reaction system, a stirring tank flow system, a flow system, a tubular reaction system, and a reactor obtained by appropriately combining these systems.

### 2. Step (2)

The method for producing a glycolic acid salt according to the present embodiment may further include step (2) of reacting formaldehyde with at least one compound selected from the group consisting of hydrogen cyanide and a cyanide in the presence of at least one compound selected from the group consisting of a metal oxide and a base to obtain glycolonitrile before step (1) described above is performed.

Step (2) is preferably, for example, a mode in which formaldehyde is reacted with hydrogen cyanide to obtain glycolonitrile. According to such a mode, it is possible to efficiently obtain glycolonitrile, and it is also possible to increase the yield of a glycolic acid salt in step (1).

In performing step (2), formaldehyde can be supplied as a formaldehyde aqueous solution (formalin).

The "at least one compound selected from the group consisting of hydrogen cyanide and a cyanide" can be supplied in any suitable form such as a gas, a liquid, or an aqueous solution.

Hereinafter, the hydrogen cyanide, the cyanide, the metal oxide, and the base used in step (2), and reaction conditions will be described more specifically.

### (i) Hydrogen cyanide and cyanide

Specific examples of the cyanide that can be used in step (2) include ammonium cyanide, sodium cyanide, potassium cyanide, cesium cyanide, and calcium cyanide.

The at least one compound selected from the group consisting of hydrogen cyanide and a cyanide is preferably hydrogen cyanide, sodium cyanide, potassium cyanide, or calcium cyanide, more preferably hydrogen cyanide, sodium cyanide, or potassium cyanide, and still more preferably hydrogen cyanide from a viewpoint of the efficiency of the reaction for producing glycolonitrile.

### (ii) Metal oxide and base

Step (2) is performed in the presence of at least one compound selected from the group consisting of a metal oxide and a base.

The at least one compound selected from the group consisting of a metal oxide and a base is preferably a hydroxide of an alkali metal, a carbonate of an alkali metal, a hydroxide of an alkaline earth metal, a carbonate of an alkaline earth metal, or a metal oxide containing 50% by mass or more of at least one element selected from the group consisting of a rare earth element, a group 4 element of the periodic table, and a group 12 element of the periodic table, and more preferably sodium hydroxide, potassium hydroxide, or a metal oxide containing 50% by mass or more of at least one element selected from the group consisting of a rare earth element, a group 4 element of the periodic table, and a group 12 element of the periodic table.

The metal oxide used in step (2) may be the same (the same kind) as or different from the metal oxide used in step (1). When steps (1) and (2) are performed continuously in a single reactor, it is preferable to use the same metal oxide in steps (1) and (2) .

Examples of the metal oxide include a metal oxide containing 50% by mass or more of at least one element selected from the group consisting of a rare earth element, a group 4 element of the periodic table, and a group 12 element of the periodic table.

Specific examples of the metal oxide include cerium oxide and lanthanum oxide as oxides of a rare earth element, zirconium oxide and titanium oxide as oxides of a group 4 element of the periodic table, and zinc oxide as an oxide of a group 12 element of the periodic table.

The metal oxide is preferably cerium oxide, zirconium oxide, or zinc oxide from a viewpoint of the yield of a glycolic acid salt, and is more preferably cerium oxide because the yield of a glycolic acid salt can be remarkably increased.

Examples of the base include ammonia, a water-soluble salt of an alkali metal, and a water-soluble salt of an alkaline earth metal.

The metal oxide used in step (2) may be the same (the same kind) as or different from the base used in step (1). When steps (1) and (2) are performed continuously in a single reactor, it is preferable to use the same base in steps (1) and (2).

Examples of the water-soluble salt of an alkali metal include a hydroxide, a halide, a carbonate, a sulfite, an acidic sulfite, a sulfate, and a carboxylate of an alkali metal.

Examples of the water-soluble salt of an alkaline earth metal include a hydroxide, a halide, a carbonate, a sulfite, an acidic sulfite, a sulfate, and a carboxylate of an alkaline earth metal.

The base is preferably a hydroxide of an alkali metal, a carbonate of an alkali metal, a hydroxide of an alkaline earth metal, or a carbonate of an alkaline earth metal, and more preferably sodium hydroxide or potassium hydroxide.

### (iii) Reaction conditions

The use amount (molar amount) of formaldehyde is preferably 0.5 to 2, more preferably 0.8 to 1.2, and still more preferably 0.95 to 1.10 with respect to the use amount (molar amount) of at least one compound selected from the group consisting of hydrogen cyanide and a cyanide.

The use amount of the at least one compound selected from the group consisting of a metal oxide and a base is usually 0.00001 to 1000 parts by mass, and preferably 0.001 to 100 parts by mass with respect to 100 parts by mass of the at least one compound selected from the group consisting of hydrogen cyanide and a cyanide.

Reaction time in step (2) only needs to be determined in consideration of the amount of a catalyst to be added and reaction temperature. In a case of a stirring tank flow system, the reaction time is preferably 10 to 300 minutes, more preferably 10 to 50 minutes, and still more preferably in a range of 15 to 40 minutes. In a case of a flow system or a tubular reaction system, the reaction time is preferably 10 to 300 minutes, more preferably 10 to 50 minutes, and still more preferably in a range of 15 to 40 minutes. When steps (1) and (2) are performed in a single reactor, the reaction time can be determined based on the yield in step (1).

The reaction temperature only needs to be determined in consideration of the above addition amount of the catalyst and the above reaction time. The reaction temperature is preferably in a range of 20 to 100°C, and more preferably in a range of 40 to 90°C.

Reaction pressure is preferably in a range of 0 to 1.0 MPa/G.

In step (2), one kind of base may be used singly, or two or more kinds thereof may be used in combination. The use amount of the base is usually 0.001 to 100000 parts by mass, and preferably 0.01 to 10000 parts by mass with respect to 100 parts by mass of the metal oxide. The molar use amount of the base is usually in a range of 0.01 to 4 with respect to the molar use amount of the at least one compound selected from the group consisting of hydrogen cyanide and a cyanide.

Examples of a reactor applicable to step (2) include a stirring tank flow system, a flow system, a tubular reaction system, and a reactor obtained by appropriately combining these systems.

Steps (1) and (2) described above may be performed individually in separate reactors, or may be performed in a single reactor. Steps (1) and (2) are preferably performed in a single reactor. Steps (1) and (2) can also be performed continuously in a single reactor.

In step (2), for example, at least one compound selected from the group consisting of hydrogen cyanide and a cyanide may be absorbed into a formaldehyde aqueous solution in an absorption tank (reactor) of a stirring tank flow system, or an absorption solution which is an aqueous solution in which at least one compound selected from the group consisting of hydrogen cyanide and a cyanide is absorbed into pure water in an absorption tank may be prepared, and then the absorption solution may be mixed with formaldehyde (aqueous solution). In addition, hydrogen cyanide and a formaldehyde aqueous solution may be added to a batch type reactor, or hydrogen cyanide and a formaldehyde aqueous solution may be added to a flow type tubular reactor.

The at least one compound selected from the group consisting of a metal oxide and a base may be formed into an aqueous solution in advance, and added to an absorption solution or a formaldehyde aqueous solution in an absorption tank.

### 3. Step (3)

A method for producing glycolic acid according to the present embodiment includes step (3) of obtaining glycolic acid from a glycolic acid salt obtained by the above-described method for producing a glycolic acid salt.

That is, in the method for producing glycolic acid according to the present embodiment, the method for producing a glycolic acid salt, including the step (1) or the steps (1) and (2), is performed, and then step (3) of obtaining glycolic acid from a glycolic acid salt obtained by the method for producing a glycolic acid salt is performed.

Examples of step (3) include: a method for directly adding an acid (for example, 10% by mass hydrochloric acid) to a reactor in which the step (1) or the steps (1) and (2) have been performed and thereby adjusting the pH to an acidic pH (for example, about pH 3.8) to obtain glycolic acid from a glycolic acid salt; a method for bringing an aqueous solution of a resulting glycolic acid salt into contact with a hydrogen ion type cation exchange resin; a method for once converting a glycolic acid salt into an ester compound, separating the ester compound, and then hydrolyzing the ester compound to obtain glycolic acid; and a method for obtaining glycolic acid by an electrodialysis method.

Among these methods, step (3) is preferably a step of directly adding an acid to a reactor in which the step (1) or the steps (1) and (2) have been performed from a viewpoint of simplifying the step. A method for obtaining glycolic acid by an electrodialysis method is preferably adopted from a viewpoint of reducing the amount of waste such as a salt.

When step (3) is a step of directly adding an acid to a reactor in which the step (1) or the steps (1) and (2) have been performed, examples of the acid that can be used include hydrochloric acid, sulfuric acid, acetic acid, and carbonic acid. As the acid, hydrochloric acid is preferably used.

When the method using a hydrogen ion type cation exchange resin is adopted in step (3), a weakly acidic cation exchange resin or a strongly acidic cation exchange resin can be used as the hydrogen ion type cation exchange resin.

When an unused hydrogen ion type cation exchange resin is used in step (3), it is preferable to sufficiently perform pretreatment and water washing of the hydrogen ion type cation exchange resin before use. Specifically, the pretreatment of the hydrogen ion type cation exchange resin can be performed by alternately washing the hydrogen ion type cation exchange resin with an acid and a base.

When a hydrogen ion type cation exchange resin applied once is regenerated and reused in step (3), the hydrogen ion type cation exchange resin can be regenerated by, for example, treating the hydrogen ion type cation exchange resin with sulfuric acid, hydrochloric acid, nitric acid, or the like, and in this case, the hydrogen ion type cation exchange resin is preferably treated with sulfuric acid. Specifically, the hydrogen ion type cation exchange resin can be regenerated by, for example, causing sulfuric acid to pass through the hydrogen ion type cation exchange resin and replacing an acid remaining in the liquid with pure water.

Treatment time in a case of using a hydrogen ion type cation exchange resin is preferably 3 to 60 minutes, and more preferably 6 to 30 minutes in a case of a batch type.

When treatment is performed continuously using a hydrogen ion type cation exchange resin, a liquid flow rate is preferably in a range of 0.1 to 100, and more preferably in a range of 1 to 10 in terms of a liquid space velocity ((L/Hr) L-cation exchange resin).

When a hydrogen ion type cation exchange resin is used, treatment temperature is preferably in a range of 5 to 70°C, and more preferably in a range of 20 to 50°C.

As a method for once converting a glycolic acid salt into an ester compound, separating the ester compound, and then hydrolyzing the ester compound to obtain glycolic acid, a conventionally known method can be used.

Examples of the electrodialysis method include a two-chamber electrodialysis method using a bipolar membrane and an anion exchange membrane or a cation exchange membrane, and a three-chamber electrodialysis method using a bipolar membrane, an anion exchange membrane, and a cation exchange membrane.

As an electrode of an electrodialysis apparatus used in the electrodialysis method, a conventionally known electrode can be used without any limitation.

Examples of a material of an anode of the electrodialysis apparatus include platinum, titanium/platinum, carbon, nickel, ruthenium/titanium, and iridium/titanium. Examples of a material of a cathode include iron, nickel, platinum, titanium/platinum, carbon, and stainless steel.

The bipolar membrane that can be used in the electrodialysis method is not particularly limited. As the bipolar membrane, a conventionally known bipolar membrane, for example, a bipolar membrane having a laminated structure in which a cation exchange membrane and an anion exchange membrane are bonded to each other can be used.

A cation exchange group that can be included in the cation exchange membrane constituting the bipolar membrane is not particularly limited. Examples of the cation exchange group include a sulfo group and a carboxy group. The cation exchange group is preferably a sulfo group.

An anion exchange group of the anion exchange membrane is not particularly limited. Examples of the anion exchange group include an ammonium group, a pyridinium group, a primary amino group, a secondary amino group, and a tertiary amino group. The anion exchange group is preferably an ammonium group.

The cation exchange membrane that can be used in the electrodialysis method is not particularly limited. As the cation exchange membrane, a conventionally known cation exchange membrane can be used. As the cation exchange membrane, for example, a cation exchange membrane in which a sulfo group, a carboxy group, and a plurality of ion exchange groups thereof are mixed can be used.

The anion exchange membrane that can be used in the electrodialysis method is not particularly limited. As the anion exchange membrane, a conventionally known anion exchange membrane can be used. As the anion exchange membrane, for example, a cation exchange membrane in which an ammonium group, a pyridinium group, a primary amino group, a secondary amino group, a tertiary amino group, and a plurality of ion exchange groups thereof are mixed can be used.

Temperature in the electrodialysis method is preferably in a range of 5 to 70°C, and more preferably in a range of 20 to 50°C.

A current density in the electrodialysis method is not particularly limited. The current density is preferably in a range of 0.1 to 100 A/dm², and more preferably in a range of 2 to 20 A/dm². A membrane spacing of the ion exchange membrane can be a commonly applied spacing. The membrane spacing of the ion exchange membrane is preferably in a range of 0.01 to 10 mm, and more preferably in a range of 0.05 to 1.50 mm.

Glycolic acid obtained in step (3) can be directly applied to a desired use without being further purified.

Glycolic acid obtained in step (3) may be further subjected to an isolation and purification treatment using a microfiltration membrane (MF), an ultrafiltration membrane (UF), an adsorbent such as activated carbon, or an anion exchange resin alone or in combination thereof, and glycolic acid purified by further performing a treatment such as removing moisture contained in the resulting glycolic acid to concentrate the glycolic acid can also be applied to a desired use.

Glycolic acid obtained from a glycolic acid salt produced by the production method of the present embodiment can be suitably applied to various uses such as a raw material for further producing a compound, cosmetics, pharmaceuticals, a boiler compound, and a detergent.

### EXAMPLES

Hereinafter, the present invention will be described more specifically using Examples and Comparative Examples. The present invention is not limited to the following Examples.

### <Example 1>

100 parts by mass of cerium oxide (trade name: cerium oxide HS manufactured by Daiichi Kigenso Kagaku Kogyo Co., Ltd.), 430 parts by mass of water, and 200 parts by mass of a 20% by mass potassium hydroxide aqueous solution were mixed to obtain a mixed solution. To the mixed solution being stirred while being maintained at a temperature of 20°C or lower, 160 parts by mass of a 52% by mass glycolonitrile aqueous solution (manufactured by Tokyo Chemical Industry Co., Ltd.) was added.

Furthermore, the resulting mixed solution was heated to 50°C while being stirred, and reacted for one hour while being maintained at 50°C to obtain a reaction solution. The resulting reaction solution was cooled to 15°C and then filtered with suction to be separated into a solid and a liquid. The resulting liquid and a washing solution obtained by washing the resulting solid with water were mixed to obtain a mixed solution. The resulting mixed solution had a pH of 10.1.

The resulting mixed solution was analyzed by high performance liquid chromatography (high performance liquid chromatography apparatus: LC-10 manufactured by Shimadzu Corporation, column: Imtakt Scherzo SS-C18, column temperature: 35°C, UV detector (wavelength: 220 nm), eluent: 0.75% by mass phosphoric acid aqueous solution, eluent supply rate: 0.5 mL/min). As a result, the yield of the resulting glycolic acid salt was 80% based on glycolonitrile.

A solution obtained by adding 10% by mass hydrochloric acid (manufactured by Wako Pure Chemical Industries, Ltd.) to the resulting mixed solution and adjusting the pH to 4.3 was analyzed by gas chromatography analysis (gas chromatograph apparatus: 7890A manufactured by Agilent Technologies, column: DB-WAX) and mass spectrum analysis (mass spectrometer: 5975C manufactured by Agilent Technologies).

As a result, since peaks (normalized peak intensity ratios corresponding to m/z values) of a mass spectrum indicated in Table 1 below were observed, it was possible to confirm that glycolic acid was obtained. In addition, since peaks of a mass spectrum indicated in Table 2 below were observed, it was also possible to confirm that glycolamide was produced as in the following scheme. Implementation conditions and results are also indicated in Table 6 below.

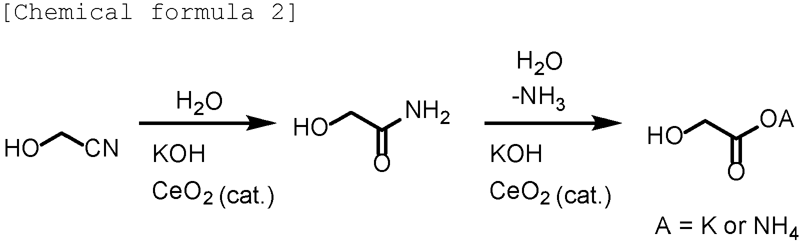

**[Table 1]**

| m/z | Peak intensity ratio | m/z | Peak intensity ratio |
|---|---|---|---|
| 29 | 40.5 | 45 | 27.1 |
| 30 | 7.1 | 46 | 2.3 |
| 31 | 100.0 | 47 | 1.1 |
| 33 | 0.6 | 49 | 0.1 |
| 40 | 0.5 | 56 | 1.3 |
| 41 | 1.8 | 58 | 1.2 |
| 42 | 4.8 | 59 | 0.7 |
| 44 | 3.7 | 77 | 0.3 |

**[Table 2]**

| m/z | Peak intensity ratio |
|---|---|
| 31 | 33.4 |
| 32 | 6.5 |
| 44 | 100.0 |
| 45 | 20.1 |
| 46 | 44.0 |
| 75 | 19.4 |
| 76 | 0.6 |

### <Example 2>

Reaction was performed as in the following scheme in a similar manner to Example 1 except that "430 parts by mass of water" in Example 1 was changed to "270 parts by mass of water", and "160 parts by mass of a 52% by mass glycolonitrile aqueous solution" was changed to "360 parts by weight of a 30% by mass glycolamide aqueous solution" (glycolamide: manufactured by Sigma-Aldrich), and solid-liquid separation was further performed to obtain a mixed solution. The resulting mixed solution had a pH of 10.1.

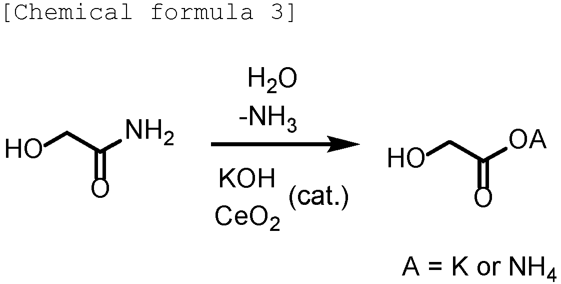

The resulting mixed solution was analyzed by high performance liquid chromatography in a similar manner to Example 1. As a result, the yield of the resulting glycolic acid salt was 81% based on glycolamide.

To the resulting mixed solution, 10% by mass hydrochloric acid (manufactured by FUJIFILM Wako Pure Chemical Industries, Ltd.) was added, and the pH was adjusted to 3.3. The resulting solution was analyzed by gas chromatography analysis and mass spectrum analysis in a similar manner to Example 1.

As a result, since peaks of a mass spectrum indicated in Table 3 below were observed, it was possible to confirm that glycolic acid was obtained. In addition, it was found that the glycolamide produced in Example 1 was a reaction intermediate in the reaction from glycolonitrile to a glycolic acid salt. Implementation conditions and results are also indicated in Table 6 below.

**[Table 3]**

| m/z | Peak intensity ratio | m/z | Peak intensity ratio |
|---|---|---|---|
| 29 | 40.8 | 45 | 27.5 |
| 30 | 7.1 | 46 | 2.4 |
| 31 | 100.0 | 47 | 2.9 |
| 33 | 0.6 | 49 | 0.2 |
| 40 | 0.5 | 56 | 1.3 |
| 41 | 1.9 | 58 | 1.6 |
| 42 | 5.1 | 59 | 0.9 |
| 44 | 4.0 | 77 | 0.2 |

### <Example 3>

100 parts by mass of cerium oxide (trade name: cerium oxide HS manufactured by Daiichi Kigenso Kagaku Kogyo Co., Ltd.), 430 parts by mass of water, and 200 parts by mass of a 20% by mass potassium hydroxide aqueous solution were mixed to obtain a mixed solution. To the mixed solution being stirred while being maintained at a temperature of 10°C or lower, 40 parts by mass of hydrogen cyanide was added. To the mixed solution being stirred while being maintained at a temperature of 15°C or lower, 120 parts by mass of a 37% by mass formaldehyde aqueous solution (reagent special grade, manufactured by FUJIFILM Wako Pure Chemical Corporation) was added. The resulting mixed solution was heated to 50°C while being stirred, and reacted for one hour as in the following scheme while being maintained at 50°C to obtain a reaction solution. The resulting reaction solution was cooled to 15°C and then filtered with suction to be separated into a solid and a liquid. The resulting liquid and a washing solution obtained by washing the resulting solid with water were mixed to obtain a mixed solution. The resulting mixed solution had a pH of 9.8. Implementation conditions and results are also indicated in Table 6 below.

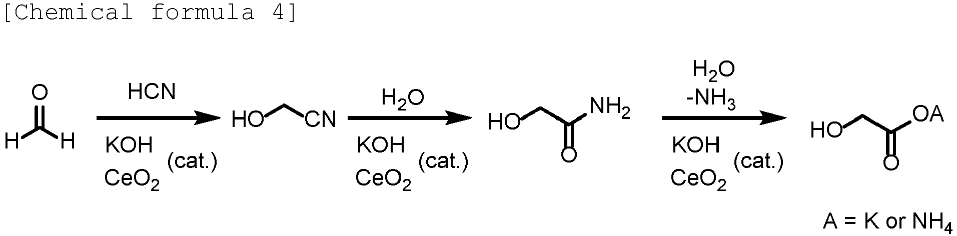

The resulting mixed solution was analyzed by high performance liquid chromatography in a similar manner to Example 1. As a result, the yield of the resulting glycolic acid salt was 79% based on formaldehyde.

A solution obtained by adding 10% by mass hydrochloric acid (manufactured by Wako Pure Chemical Industries, Ltd.) to the resulting mixed solution and adjusting the pH to 3.8 was analyzed by gas chromatography analysis and mass spectrum analysis in a similar manner to Example 1.

As a result, since peaks of a mass spectrum indicated in Table 4 below were observed, it was possible to confirm that glycolic acid was obtained. In addition, since peaks of a mass spectrum indicated in Table 5 below were observed, it was also possible to confirm that glycolamide was produced as a reaction intermediate. In addition, from the results of Example 3, it was found that the produced glycolamide was a reaction intermediate in the reaction from formaldehyde and hydrogen cyanide to a glycolic acid salt. Implementation conditions and results are also indicated in Table 6 below.

**[Table 4]**

| m/z | Peak intensity ratio | m/z | Peak intensity ratio |
|---|---|---|---|
| 29 | 41.7 | 45 | 27.5 |
| 30 | 7.4 | 46 | 2.4 |
| 31 | 100.0 | 47 | 2.9 |
| 33 | 0.6 | 49 | 0.3 |
| 40 | 0.5 | 56 | 1.4 |
| 41 | 2.0 | 58 | 1.4 |
| 42 | 5.1 | 59 | 0.8 |
| 44 | 3.8 | 77 | 0.2 |

**[Table 5]**

| m/z | Peak intensity ratio |
|---|---|
| 31 | 30.6 |
| 32 | 3.5 |
| 44 | 100.0 |
| 45 | 14.5 |
| 46 | 47.4 |
| 75 | 19.0 |
| 76 | 0.7 |

### <Example 4>

A mixed solution was obtained in a similar manner to Example 3 except that the mixed solution obtained by adding a formaldehyde aqueous solution in Example 3 was heated to 70°C and reacted at 70°C for four hours.

The resulting mixed solution was analyzed by high performance liquid chromatography in a similar manner to Example 1. As a result, the yield of the resulting glycolic acid salt was 99% based on formaldehyde. Implementation conditions and results are also indicated in Table 6 below.

### <Example 5>

A mixed solution was obtained in a similar manner to Example 3 except that zirconium oxide (trade name: RC-100 zirconium oxide, manufactured by Daiichi Kigenso Kagaku Kogyo Co., Ltd.) was used instead of cerium oxide in Example 3.

As a result of analyzing the resulting mixed solution by high performance liquid chromatography in a similar manner to Example 1, the yield of the resulting glycolic acid salt was 23% based on formaldehyde. Implementation conditions and results are also indicated in Table 7 below.

### <Example 6>

A mixed solution was obtained in a similar manner to Example 3 except that zinc oxide (trade name: FINEX-30, manufactured by Sakai Chemical Industry Co., Ltd.) was used instead of cerium oxide in Example 3.

The resulting mixed solution was analyzed by high performance liquid chromatography in a similar manner to Example 1. As a result, the yield of the resulting glycolic acid salt was 19% based on formaldehyde. Implementation conditions and results are also indicated in Table 7 below.

### <Example 7>

A mixed solution was obtained in a similar manner to Example 3 except that "430 parts by mass of water" was changed to "470 parts by mass of water" and "200 parts by mass of a 20% by mass potassium hydroxide aqueous solution" was changed to "150 parts by mass of a 20% by mass sodium hydroxide aqueous solution" in Example 3.

The resulting mixed solution was analyzed by high performance liquid chromatography in a similar manner to Example 1. As a result, the yield of the resulting glycolic acid salt was 84% based on formaldehyde. Implementation conditions and results are also indicated in Table 7 below.

**[Table 6]**

| | Example 1 | Example 2 | Example 3 | Example 4 |
|---|---|---|---|---|
| Raw material | Glycolonitrile Water | Glycolamide Water | Formaldehyde Water Hydrogen cyanide | Formaldehyde Water Hydrogen cyanide |
| Metal oxide | Cerium oxide | Cerium oxide | Cerium oxide | Cerium oxide |
| Base | Potassium hydroxide | Potassium hydroxide | Potassium hydroxide | Potassium hydroxide |
| Reaction temperature (°C) | 50 | 50 | 50 | 70 |
| Reaction time (Hour) | 1 | 1 | 1 | 4 |
| Yield of glycolic acid salt (%) | 80 (Based on glycolonitrile) | 81 (Based on glycolamide) | 79 (Based on formaldehyde) | 99 (Based on formaldehyde) |

**[Table 7]**

| | Example 5 | Example 6 | Example 7 |
|---|---|---|---|
| Raw material | Formaldehyde Water Hydrogen cyanide | Formaldehyde Water Hydrogen cyanide | Formaldehyde Water Hydrogen cyanide |
| Metal oxide | Zirconium oxide | Zinc oxide | Cerium oxide |
| Base | Potassium hydroxide | Potassium hydroxide | Sodium hydroxide |
| Reaction temperature (°C) | 50 | 50 | 50 |
| Reaction time (Hour) | 1 | 1 | 1 |
| Yield of glycolic acid salt (%) | 23 (Based on formaldehyde) | 19 (Based on formaldehyde) | 84 (Based on formaldehyde) |

### INDUSTRIAL APPLICABILITY

The method for producing a glycolic acid salt according to the present invention makes it possible to efficiently produce a glycolic acid salt that can be a raw material (intermediate product) for producing glycolic acid by a simpler process.

## Claims

1. A method for producing a glycolic acid salt, comprising step (1) of reacting at least one compound selected from the group consisting of glycolonitrile and glycolamide with water in the presence of a metal oxide containing 50% by mass or more of at least one element selected from the group consisting of a rare earth element, a group 4 element of the periodic table, and a group 12 element of the periodic table, and a base to obtain a glycolic acid salt.

2. The method for producing a glycolic acid salt according to claim 1, further comprising
step (2) of reacting formaldehyde with at least one compound selected from the group consisting of hydrogen cyanide and a cyanide in the presence of at least one compound selected from the group consisting of a metal oxide and a base to obtain glycolonitrile
before the step (1) is performed.

3. The method for producing a glycolic acid salt according to claim 2, wherein the steps (1) and (2) are performed using a single reactor.

4. The method for producing a glycolic acid salt according to any one of claims 1 to 3, wherein the metal oxide is at least one metal oxide selected from the group consisting of cerium oxide, zinc oxide, zirconium oxide, and a composite oxide in which two or more of cerium oxide, zinc oxide, and zirconium oxide are combined.

5. The method for producing a glycolic acid salt according to any one of claims 1 to 4, wherein the metal oxide is cerium oxide.

6. The method for producing a glycolic acid salt according to any one of claims 1 to 5, wherein the base is at least one base selected from the group consisting of an alkali metal hydroxide and an alkaline earth metal hydroxide.

7. The method for producing a glycolic acid salt according to any one of claims 1 to 6, wherein the base is potassium hydroxide or sodium hydroxide.

8. The method for producing a glycolic acid salt according to claim 2, wherein in the step (2), the formaldehyde is reacted with the hydrogen cyanide.

9. A method for producing glycolic acid, comprising step (3) of obtaining glycolic acid from a glycolic acid salt obtained by the method for producing a glycolic acid salt according to any one of claims 1 to 8.
